# EUROPEAN PATENT APPLICATION

(11) **EP 2 380 542 A1**
(43) Date of publication of application: **26.10.2011**
(21) Application number: 09835108.3
(22) Date of filing: 24.12.2009
(51) Int. Cl.: A61F 13/15, A61F 13/53, A61F 13/539

(54) **ABSORBENT ARTICLE**

(30) Priority: 25.12.2008 JP 2008331022
(71) Applicant: Unicharm Corporation, Shikokuchuo-shi, Ehime 799-0111 (JP)
(72) Inventor: KURODA, Kenichiro, Kanonji-shi Kagawa 769-1602 (JP); NODA, Yuki, Kanonji-shi Kagawa 769-1602 (JP)
(74) Representative: Eke, Philippa Dianne
(86) International application number: PCT/JP2009/071859
(87) International publication number: WO 2010/074319

(57) **Abstract**

In an absorbent article, to keep a body fluid contacted with a compressed groove from diffusing to the end part of the compressed groove and prevent the absorbent article from deforming due to contact with a body fluid.

An absorbent article comprising at least a liquid-permeable sheet, a liquid-impermeable sheet, and an absorption body sandwiched between the liquid-permeable sheet and the liquid-impermeable sheet, which is an absorbent article where, on the skin-contact surface side, a pair of compressed grooves for integrating from the liquid-permeable sheet to the absorption body are formed along the longitudinal direction, wherein
the compressed groove consists of a high-compressed part and a low-compressed part, the high-compressed part is composed of nearly transverse high-compressed parts formed to almost fully traverse the compressed groove in the widthwise direction and disposed at intervals in the longitudinal direction of the compressed groove and non-transverse high-compressed parts formed not to traverse the compressed groove and disposed at intervals in the region between nearly transverse high-compressed parts, and by the arrangement of the non-transverse high-compressed parts, the low-compressed part is formed to continue in the longitudinal direction of the compressed groove between nearly transverse high-compressed parts.

## Description

### TECHNICAL FIELD

The present invention relates to an absorbent article used for sanitary napkins, pantiliners, incontinence pads and the like, which allows for no diffusion of a body fluid and ensures deformation stability of the absorbent article when wetted.

### BACKGROUND ART

Japanese Unexamined Patent Publication (Kokai) No. 2004-321393 discloses an absorbent article having, on the skin-contact surface side, a groove consisting of a high-compressed part and a low-compressed part, wherein the high-compressed parts are formed along the side edge of the groove by shifting the position from each other in the longitudinal direction of the groove and the low-compressed part is continued in the length direction of the groove. Also, for example, in Kokai Nos. 2003-265518 and 2003-38555, an absorbent article with a groove having the same structure is disclosed. The groove having such a structure is provided for the purpose of reducing leakage in the lateral direction by forming a low-compressed part continuously in the longitudinal direction of the groove and thereby appropriately diffusing a body fluid in the longitudinal direction.

However, since in these absorbent articles, the low-compressed part is formed almost over the entire region of the compressed groove, and therefore a body fluid contacted with the groove may diffuse to the front and rear end parts of the groove. In turn, the high- and low-compressed parts contacted with a body fluid are wetted and the joining force between constituent layers of the absorbent article is weakened, which not only induces twisting due to movement but also causes breakage of bonding between pulps as the constituent material of the absorption body, leading to deterioration in the rigidity of the absorbent article. On the other hand, the portion kept from contact with a body fluid has high rigidity, and therefore does not follow the body during wearing, and this gives an uncomfortable feeling.

### SUMMARY OF THE INVENTION

An object of the present invention is to keep a body fluid contacted with a compressed groove from diffusing to the end part of the compressed groove and to prevent the absorbent article from deforming due to contact with a body fluid.

Under these circumstances, the present inventors have made intensive studies; as a result, it has been found that when transverse high-compressed parts almost fully traversing the compressed groove are disposed at intervals, and furthermore non-transverse high-compressed parts not traversing the compressed groove are disposed at intervals in the region between transverse high-compressed parts, a low-compressed part continuing in the longitudinal direction of the compressed groove can be formed and the above-described problems can be thereby solved. The present invention has been accomplished based on this finding.

That is, (1) the present invention provides an absorbent article comprising at least a liquid-permeable sheet, a liquid-impermeable sheet, and an absorption body sandwiched between the liquid-permeable sheet and the liquid-impermeable sheet, which is an absorbent article where, on the skin-contact surface side, a pair of compressed grooves for integrating from the liquid-permeable sheet to the absorption body are formed along the longitudinal direction, wherein
the compressed groove consists of a high-compressed part and a low-compressed part, wherein the high-compressed part is composed of nearly transverse high-compressed parts formed so as to almost fully traverse the compressed groove in the widthwise direction and disposed at intervals in the longitudinal direction of the compressed groove and non-transverse high-compressed parts formed so as not to traverse the compressed groove and disposed at intervals in the region between nearly transverse high-compressed parts, and by the arrangement of the non-transverse high-compressed parts, the low-compressed part is formed so as to continue in the longitudinal direction of the compressed groove between nearly transverse high-compressed parts.
(2) The present invention provides the absorbent article according to (1), wherein at least a part of the non-transverse high-compressed parts are provided to cross the longitudinal centerline of the compressed groove.
(3) The present invention provides the absorbent article according to (1) or (2), wherein at least one of the nearly transverse high-compressed parts is provided to be symmetric with respect to the longitudinal centerline of the compressed groove.
(4) The present invention provides the absorbent article according to any one of (1) to (3), wherein the distance between nearly transverse high-compressed parts is from 10 to 50 mm.
(5) The present invention provides the absorbent article according to any one of (1) to (4), wherein the width of the compressed groove in which the nearly transverse high-compressed part is disposed is larger than the width of the compressed groove in which the nearly transverse high-compressed apart is not disposed.
(6) The present invention provides the absorbent article according to any one of (1) to (5), wherein the distance between a nearly transverse high-compressed part and a non-transverse high-compressed part located closest to the nearly transverse high-compressed part is larger than the distance between non-transverse high-compressed parts located between nearly transverse high-compressed parts.

In the absorbent article of the present invention, although a body fluid diffuses in the longitudinal direction due to the longitudinally continuing low-compressed region, such diffusion of a body fluid is stopped at predetermined portions by the nearly transverse compressed part, and therefore the body fluid can be prevented from diffusing to reach the front and rear ends of the absorbent article. Also, since diffusion of a body fluid is stopped at predetermined portions, the compressed groove region kept from contact with the body fluid can maintain a dry state.

### BRIEF DESCRIPTION OF THE DRAWINGS

Fig. 1 shows a plan view illustrating the first embodiment of the absorbent article of the present invention, and an enlarged view of the portion surrounded by a square.
Fig. 2 shows a cross-sectional view along A-A' of the absorbent article of Fig. 1, and an enlarged perspective view of the right compressed groove.
Fig. 3 shows a plan view illustrating the second embodiment of the absorbent article of the present invention, and an enlarged view of the portion surrounded by a square.
Fit. 4 shows variations of the compressed groove and an enlarged view of the portion surrounded by a square.
Fig. 5 shows a plan view illustrating the third embodiment of the absorbent article of the present invention, and an enlarged view of the portion surrounded by a square.
Fig. 6 shows a plan view illustrating the fourth embodiment of the absorbent article of the present invention, an enlarged view of the front end part of the rear-side compressed groove, and an enlarged view of the portion surrounded by a square.

### MODE FOR CARRYING OUT THE INVENTION

A preferred embodiment (first embodiment) of the absorbent article of the present invention is described below by referring to the drawings.

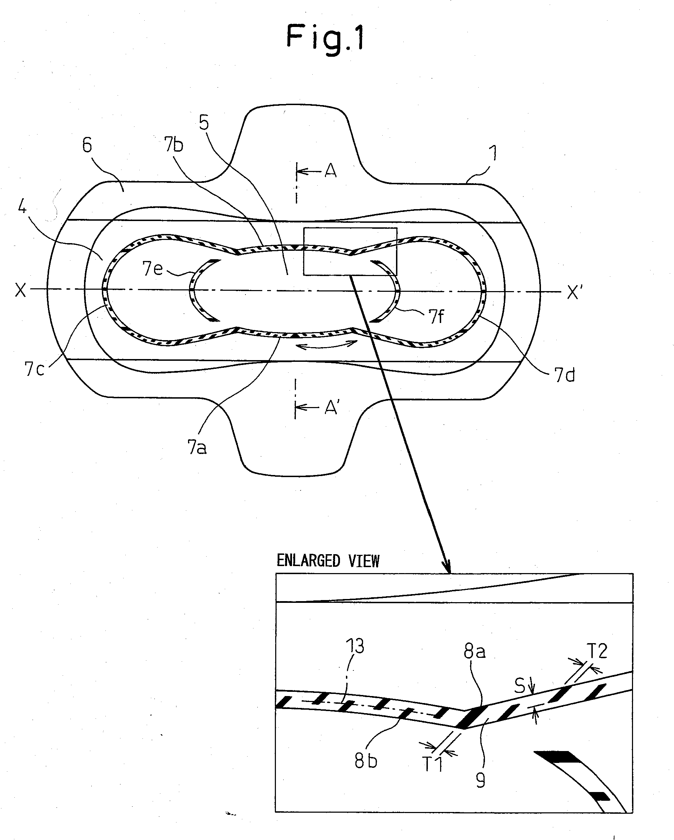

As shown in Fig. 1, the absorbent article 1 of the first embodiment comprises at least a liquid-permeable sheet 2, a liquid-impermeable sheet 3, and an absorption body 4 sandwiched between the liquid-permeable sheet and the liquid-impermeable sheet.

In the first embodiment, on the skin-contact surface side of the absorbent article, right and left compressed grooves 7a and 7b located in the longitudinally-extending side edge part of the center region 5 of the absorption body are formed, a front-side compressed groove 7c located in the longitudinally-extending front part of the side edge part is formed, and a rear-side compressed groove 7d located in the longitudinally-extending rear part of the side edge part, is formed. Furthermore, transverse compressed grooves 7e and 7f traversing the longitudinal centerline (X-X') are formed so as to surround the center region 5 of the absorption body. The compressed groove fulfills a role of, not only integrating the liquid-permeable sheet and the absorption body, but also stopping lateral spreading of a body fluid and allowing the body fluid to be efficiently transferred beneath the absorption body.

As shown in the enlarged view (zoomed in the portion surrounded by a square) of Fig. 1, the compressed groove consists of a high-compressed part 8 and a low-compressed part 9 disposed alternating with the high-compressed part. The high-compressed part is compressed in a ratio of 90% or more based on the thickness of absorption body in the vicinity of the high-compressed part, and the low-compressed part is compressed in a ratio of 20% to less than 90%. By providing a high-compressed part and a low-compressed part, the joining force between the liquid-permeable sheet and the absorption body can be increased and the excrement can be collected in the high-compressed part to prevent diffusion of the excrement. Also, the high-compressed part is composed of nearly transverse high-compressed parts 8a formed so as to almost fully traverse the compressed groove in the width direction and disposed at intervals in the longitudinal direction of the compressed groove, and non-transverse high-compressed parts 8b formed not so as to traverse the compressed groove and disposed at intervals between nearly transverse high-compressed parts. By such arrangement of non-transverse high-compressed parts, the low-compressed part is formed so as to continue in the longitudinal direction of the compressed groove between nearly transverse high-compressed parts. Therefore, in the absorbent article of the present invention, the absorption rate of a body fluid can be maintained and, at the same time, the diffusion range of the body fluid can be controlled. Also, the continuous low-compressed part is divided by the nearly transverse high-compressed part, and a rigidity difference is thereby produced near the boundary with the nearly transverse compressed part in the compressed groove, which makes it easy to start folding therefrom and can enhance the flexibility of the absorbent article.

The width of the compressed groove is uniform and may be from 1 to 5 mm. If the width is less than 1 mm, the entire absorbent article is too low in rigidity and therefore twisting may be generated, whereas if it exceeds 5 mm, the absorbent article can hardly fit to the body due to excessively high rigidity.

The term "nearly transverse high-compressed part" refers to a high-compressed part formed so as to almost fully traverse the compressed groove in the width direction, and the term "almost" as used herein means that the high-compressed part traverses at least 80%, preferably at least 90%, of the compressed groove. If the ratio is less than 80%, the region where the low-compressed part is continuously formed cannot be sufficiently divided (stopped). Also, if it is less than 80%, the low-compressed part continues over the entire compressed groove, allowing for the presence of a rigid region, and the adherence to the body is impaired. At least one of the nearly transverse high-compressed parts is preferably provided to be symmetric with respect to the longitudinal centerline of the compressed groove. Therefore, the flexibility of the absorbent article can be enhanced.

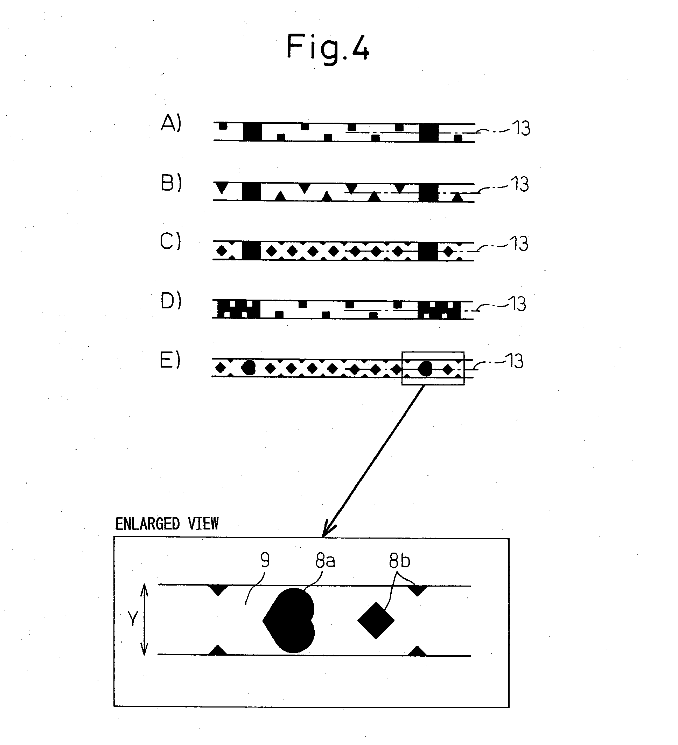

The shape of the nearly transverse high-compressed part is not particularly limited, as long as it is a shape capable of almost fully traversing the compressed groove in the width direction to completely stop the body fluid flow at that position or a shape capable of changing the rigidity of the low-compressed part continuously extending in the longitudinal direction. For example, the shape may be, in planar view, a heart shape (Fig. 4), a box shape, a diamond shape, a triangular shape or a dot shape. The nearly transverse high-compressed parts are disposed at constant intervals in the compressed groove and may differ in the shape from each other. In the first embodiment, the shape of the nearly transverse high-compressed part is substantially a parallelogram.

Although it varies depending on the shape of the nearly transverse high-compressed part, the longitudinal length (T1) of the nearly transverse high-compressed part in the compressed groove is preferably from 0.5 to 5 mm at a maximum in the longitudinal direction of the shape.

The widthwise length (S) of the non-transverse high-compressed part 8b in the compressed groove is not particularly limited, but is, for example, from 0.5 to 4 mm. In the first embodiment, the length S is a length from the outside compressed line (not shown) to a portion over the longitudinal centerline 13 of the compressed groove. Also, the length (T2) of the non-transverse high-compressed part in the longitudinal direction of the compressed groove is shorter than T1 and is, for example, preferably from 0.5 to 4 mm.

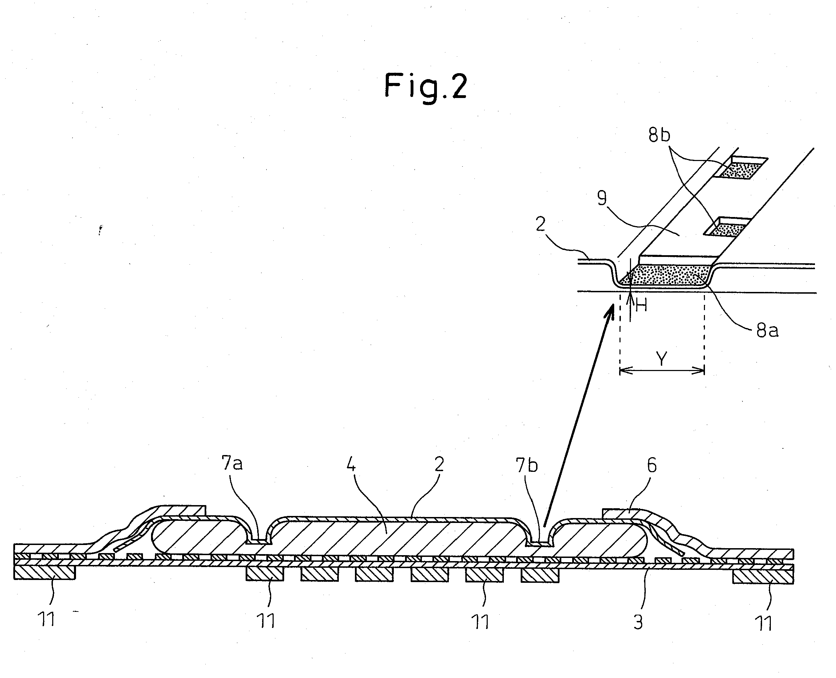

The density of the absorption body in the high-compressed part and the low-compressed part is described by referring to Fig. 2. Fig. 2 is a cross-sectional view along A-A' in Fig. 1 and is an enlarged perspective view of the right compressed groove 7b.

The density of both the nearly transverse high-compressed part and the non-transverse high-compressed part may be from 0.7 g/cm³ or more. Because, when the density is 0.7 g/cm³ or more, the compressed pulp is kept from readily recovering even when put into contact with a body fluid, and the shape of the nearly transverse high-compressed part can be easily maintained. The density of the low-compressed part is lower than the density of the high-compressed part, and is, for example, from 0.2 to 0.6 g/cm³. If the density is less than 0.2 g/cm³, the diffusibility for the body liquid is low and the body fluid runs over the groove to cause lateral leakage, whereas if it exceeds 0.6 g/cm³, not only the diffusibility by the pulp becomes extremely bad, but also the rigidity is excessively increased to hinder the absorbent article from fitting to the body. Incidentally, the density is determined by the following method. A region with a dimension of 20 mm×10 mm is cut out from the compressed groove and measured for the basis weight and the thickness (H) at a magnification of 30, and the value of thickness is divided by the value of basis weight.

The density of the nearly transverse high-compressed part and the density of the non-transverse high-compressed part may be the same or different. For example, the density of the non-transverse high-compressed part may be lower.

The second embodiment of the absorbent article of the present invention is described below. The second embodiment is described by referring mainly to the points differing from the first embodiment, and description of the same points is omitted. Accordingly, the description of the first embodiment is appropriately applied to the second embodiment.

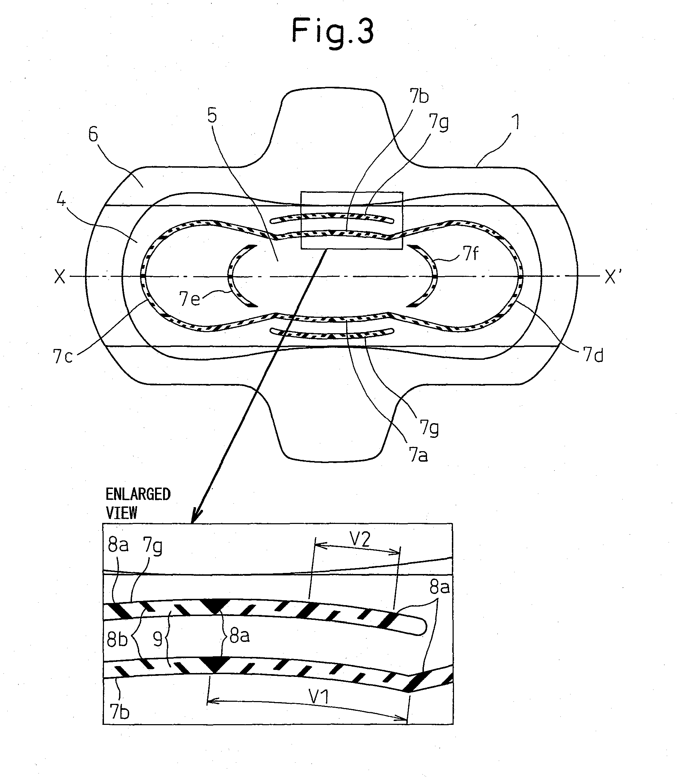

In the second embodiment, as shown in Fig. 3, a side edge-part compressed groove 7g is disposed by each of the right and left compressed grooves 7a and 7b. By further providing a side edge-part compressed groove, with respect to the leakage in the lateral direction, reduction in the leakage is brought about because the number of grooves guiding the diffusion in the longitudinal direction is increased.

The length (V1) between nearly transverse high-compressed parts may be from 10 to 50 mm so that the rigidity of the low-compressed part can be divided and while reducing the rigidity of the entire absorbent article, the body fluid coming into contact with the low-compressed part can be diffused in the front-rear direction without causing lateral leakage. If the length is less than 10 mm, the length between nearly transverse high-compressed parts is too small, failing in diffusing a sufficiently large amount of a body liquid, and lateral leakage may be caused. If the length exceeds 50 mm, the rigid region is too long and fitting to the curve of the body becomes difficult. Also, with respect to this embodiment, the length (V2) between nearly transverse high-compressed parts of the side edge-part compressed groove 7g is shorter than V1 and is, for example, shorter by around 10 mm than V1. Usually, excessive rigidity may be generated in the longitudinal direction by providing a plurality of compressed grooves, and therefore in this embodiment, the above-described dimension is employed so as to reduce the rigidity of the groove added. However, by previously setting the rigidity of the absorption body itself low, excessive rigidity may be avoided even when a plurality of grooves are provided. Therefore, the length (V2) between nearly transverse high-compressed parts of the side edge-part compressed groove 7g may be the same as V1.

Fig. 4 shows examples of the compressed groove configuration. Even by the following configurations of the compressed groove, the same effect as that in the first embodiment is obtained.

In Example A), the non-transverse high-compressed part is formed not to traverse the longitudinal centerline of the compressed groove. The low-compressed part continues in the longitudinal direction of the compressed groove, similarly to the compressed groove of the first embodiment, and therefore the body liquid diffuses between nearly transverse high-compressed parts. Also in Example D), the non-transverse high-compressed part is formed not to traverse the longitudinal centerline of the compressed groove. However, in Example D), the length (T1) of the nearly transverse high-compressed part is long compared with T1 of the compressed groove of the first embodiment or T1 in Examples A) to C) and E) shown in Fig. 4, so that the body fluid even when flows in a large amount on the surface of the low-compressed part can be stopped by the nearly transverse high-compressed part. However, when the length is too large, excessively high rigidity is created to give an uncomfortable feeling, and therefore it is preferred to reduce the rigidity in the longitudinal direction, for example, by the method of providing a partially relieving portion in the nearly transverse high-compressed part. In Examples A) and D), the low-compressed part is continuously formed on the longitudinal centerline of the compressed groove and in turn, the rigidity in the longitudinal direction is high compared with the following Examples B), C) and E), as a result, the flexibility of the absorbent article tends to be slightly poor.

On the other hand, in the first embodiment or Example B), the non-transverse high-compressed part is formed to traverse the longitudinal centerline, and therefore the rigidity in the longitudinal direction is suppressed and flexibility of the absorbent article is enhanced. Furthermore, the shape of the non-transverse high-compressed part is a triangular shape and this is advantageous in that flexible deformation is facilitated even when a force is applied to the compressed groove from an oblique direction.

In Examples C) and E), the non-transverse high-compressed parts are provided in a houndstooth check pattern. Therefore, the body fluid diffuses by snaking its way along the low-compressed region (the diffusion rate becomes low) and not only the leakage can be prevented but also even when contacted with a large amount of a body fluid, the absorbent article can be kept from being twisted.

Furthermore, the non-transverse high-compressed part is present on the longitudinal centerline of the compressed groove, whereby the rigidity of the low-compressed region is not limited in its directionality and flexibility is enhanced. Even when contacted with a large amount of a body fluid, thanks to separation of the liquid-permeable sheet from the absorption body in the low-compressed region, the absorbent article can be kept from being twisted.

These examples of the compressed groove may be used in combination. For example, Example C) or E) is used for the right and left compressed grooves and Example B) is used for the front-side compressed groove and the rear-side compressed groove.

In the third embodiment, the right or left compressed groove 7a or 7b and the front-side compressed groove 7c, which are continuously formed in the first embodiment, are separated, and the right or left compressed groove 7a or 7b and the rear-side compressed groove 7d are separated. Therefore, flexibility in the longitudinal direction of the absorbent article is enhanced. When a force is applied to the right and left compressed grooves from leg openings during wearing of the absorbent article, the force is transmitted in the direction closer to the longitudinal centerline, the absorption body on the longitudinal centerline forms a bulge part on the skin-contact surface side, and the bulge part is connected with the front end part or rear end part of the right or left compressed groove to form a projection. However, the low-compressed groove is separated and this allows for formation of a gently convex bulge part but not a projection.

The right and left compressed grooves are located to extend across the position corresponding to the excretion part, to which a force is applied from leg openings during wearing. These compressed grooves are designed to convex inwardly in the width direction in the center part (near the centerline (A-A')) and while the distance between the right and left compressed grooves is increased in the side edge part direction as they proceed toward the front or rear direction, they face each other across the longitudinal center line near the front or rear end part.

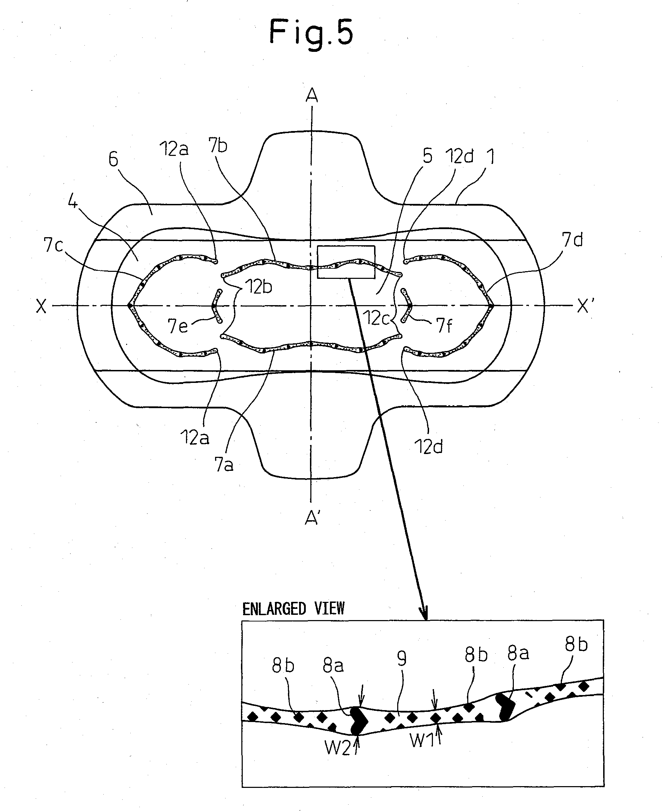

However, the right or left compressed groove 7a or 7b located on the widthwise innermost side is preferably formed such that its front end part 12b and rear end part 12c face outwardly in the width direction or run in parallel with the longitudinal centerline, so that the above-described bulge part can be formed. The bulge part reduces uncomfortable feeling due to rubbing of the skin, and the body fluid adhering on the longitudinal centerline rolls down on the slant face of the bulge part to prevent the body fluid from diffusing to cause leakage. Fig. 5 shows a configuration where the front end part 12b and the rear end part 12c are formed to face outwardly in the width direction.

The distance between front end parts of the right and left compressed grooves is preferably equal to the distance between rear end parts and shorter than the distance between right and left compressed grooves in the center part. Therefore, the force from leg openings is first transmitted to the center part and then transmitted therefrom to the front or rear end part towards which the distance between compressed grooves is decreased, and the force from leg openings can be concentrated in the front and rear end part directions. As a result, a higher bulge part can be formed in the front and rear parts across the center part than in the center part of the absorbent article and in turn, the body fluid received in the center part can be made to scarcely diffuse towards the front and rear directions.

The front-side compressed groove 7c and the rear-side compressed groove 7d are preferably disposed such that widthwise overlap is provided between the rear end part 12a of the front-side compressed groove 7c and the front end part 12b of the right and left compressed grooves 7a and 7b, and between the front end part 12d of the rear-side compressed groove 7d and the rear end part 12c of the right and left compressed grooves 7a and 7b. Even when the body fluid is absorbed in the vicinity of the rear end part of the front-side compressed groove and in the vicinity of the front end part of the rear-side compressed groove or even when the body fluid is diffused in the width direction, the leakage is reduced, because a compressed groove is further present on the widthwise outer side.

The distance between the right and left compressed grooves in the center part is preferably from 20 to 50 mm. If the distance is less than 20 mm, the absorption surface becomes narrow and leakage may occur, whereas if it exceeds 50 mm, the distance becomes larger than the width between leg openings and the force from leg openings cannot be transmitted. The difference of the distance between right and left compressed grooves in the center part from the distance between front end parts (or rear end parts) of the right and left compressed grooves is preferably from 5 to 30 mm. If the difference is less than 5 mm, the force cannot be adequately transmitted to the front and rear end parts from the center part and a sufficient effect of making the body fluid received in the center part to scarcely diffuse toward the front and rear directions can be hardly obtained. If the difference exceeds 30 mm, the distance between front end parts (or the distance between rear end parts) becomes small and a desired bulge part cannot be formed in the forward or rearward part of the center part.

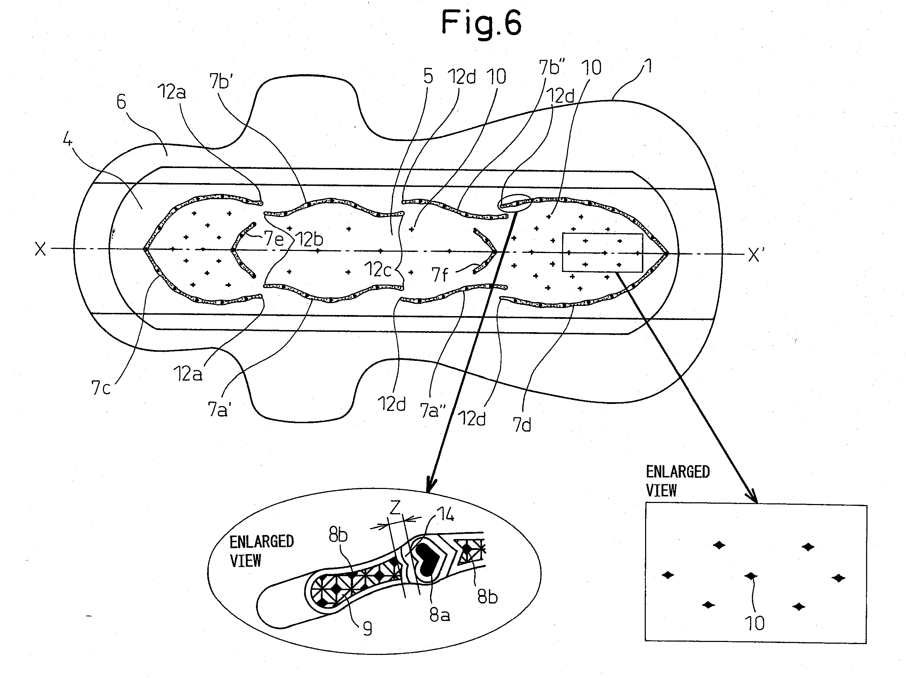

The width of the compressed groove is almost the same in the first and second embodiments. On the other hand, in the third embodiment, as shown in the enlarged view of Fig. 5, the width (W1) of the compressed groove between a nearly transverse high-compressed part region and a nearly transverse high-compressed part region is preferably smaller than the width (W2) of the compressed groove where a nearly transverse high-compressed part region is located. The same applies to the embodiment of Fig. 6 (in the enlarged view of Fig. 6, not shown). Therefore, the rigidity of the compressed groove between a nearly transverse high-compressed part region and a nearly transverse high-compressed part region can be decreased and the wearability can be more enhanced.

In the fourth embodiment, as shown in Fig. 6, each of the right and left compressed grooves 7a and 7b in Fig. 1 is further separated into two parts (7a' and 7a", and 7b' and 7b"). Out of the compressed grooves after separation into two parts, each of the right and left compressed grooves 7a' and 7b' is formed such that its front end part 12b and rear end part 12c face outwardly in the width direction or run in parallel with the longitudinal centerline.

Also, in the fourth embodiment, the transverse compressed grooves 7e and 7f may differ in the shape. The front-side transverse compressed groove 7e is in a shape of extending in the width direction, and the rear-side transverse compressed groove 7f is in a shape of extending in the longitudinal direction and takes a more sharply-angled shape. This difference in the shape produces a difference in the rigidity of the transverse compressed groove part. That is, the rigidity of the front-side transverse compressed groove part is high as compared with the rear-side transverse compressed groove part. By providing transverse compressed grooves differing in rigidity, a force applied to the right and left compressed grooves is transmitted to the front and rear end parts of the right and left compressed grooves, and the force is then transmitted to the rear-side transverse compressed groove having a lower rigidity, as a result, a higher bulge part than the bulge part formed between front end parts of the right and left compressed grooves is formed between rear end parts of the right and left compressed grooves.

In the fourth embodiment, the difference in rigidity is created by the shape of the front-side and rear-side transverse compressed grooves, but in the first to third embodiments above, the difference in rigidity may be created by adjusting the degree of embossing when providing front-side and rear-side transverse compressed grooves having the same shape. The difference in rigidity between front-side and rear-side transverse compressed grooves is preferably from 2 to 10 mN (as measured by Gurley flexibility tester (manufactured by Yasuda Seiki Seisakusho, Ltd.)). If the difference is less than 2 mN, the force cannot be efficiently transmitted to the rear side, whereas if it exceeds 10 mN, the force is unevenly transmitted to one side and the absorbent article is excessively deformed.

In the absorbent article of the fourth embodiment, even when the body fluid is leaked to the rearward of the bottom through the groove in the bottom at bedtime, the leakage can be stopped by the rear-side transverse compressed groove, and therefore this absorbent article is suitable for nighttime use.

In the fourth embodiment, for reducing the increase in the rigidity due to continuing of the low-compressed part, an intermittent part 14 is provided between a nearly transverse high-compressed part and a non-transverse high-compressed part located nearest to the nearly transverse high-compressed part, and the intermittent part is designed so as to have a width (Z) longer than the distance between non-transverse high-compressed parts located between nearly transverse high-compressed parts.

The point-like compressed part is described below.

On the skin-contact surface side of the absorbent article, as shown in Fig. 6, a plurality of point-like compressed parts 10 for integrating the permeable sheet and the absorption body may be disposed at constant intervals. By arranging point-like compressed parts, when a bulge part is formed in the absorbent article, a groove concaved toward the skin-contact surface side is formed on the slant face of the bulge part and the excrement attached to the top of the bulge part slides on the slant face and is absorbed in the groove without causing lateral leakage.

The shape of the point-like compressed part is not particularly limited and may be, for example, a box shape, a triangular shape, a diamond shape, a round shape or a star shape, but is preferably a diamond shape long in the longitudinal direction. Therefore, a point-like compressed part with a long diamond shape is liable to lift toward the skin-contact surface side when a force is applied to the right and left compressed grooves, and folding of the absorbent article is facilitated.

The width of the top-surface opening of the point-like compressed part is preferably smaller than the width of the top-surface opening of the compressed groove. Specifically, the width is from 0.3 to 5 mm, preferably from 0.5 to 3 mm, and is usually about 1.2 mm.

The distance between a compressed groove and a point-like compressed part and the distance between point-like compressed parts are 20 mm or less, preferably 15 mm or less, more preferably 10 mm or less. The depth of the point-like compressed part is preferably larger than the depth of the low-compressed part of the compressed groove.

The materials constituting the absorbent article are described below.

The absorption body may be one which absorbs and holds a body fluid, and a bulky material hardly loosing its shape and less causing chemical irritation is preferred. For example, a cellulose such as fluffed pulp and cotton, a regenerated cellulose such as rayon and fibril rayon, a semisynthetic cellulose such as acetate and triacetate, a particulate polymer, a fibrous polymer, a thermoplastic hydrophobic chemical fiber, a thermoplastic hydrophobic chemical fiber subjected to a hydrophilization treatment, and an air-laid pulp subjected to a chemical bonding treatment may be used individually or as a mixture. From the standpoint that good hydrophilicity is maintained, a cellulose fiber is preferred.

The method for forming such a material into an absorption body is not particularly limited, but other than a method of forming fluffed pulp as described in Examples 1 to 3 below, a method of forming a sheet, for example, by an air-laid process, a melt blown process, a spunlace process or a papermaking process may be used.

As the absorption body, a cellulose foam, a continuous foam of synthetic resin, or the like may be also used. Furthermore, a foam or the above-described material shaped into sheet may be used by pulverizing and then forming it into an absorption body. Among these, an absorption body having a basis weight of 100 to 2,000 g/m² and a bulkiness of 1 to 50 mm, obtained by mixing pulp in a ratio of 80 to 100% and particulate polymer in a ratio of 20 to 0%, covering the mixture with a paper backing such as tissue paper, and forming it into a sheet by embossing, may be used.

The embossing treatment is performed to prevent the absorption body from loosing its shape, and the embossing area ratio is from 10 to 100%, preferably from 30 to 80%.

Examples of the material for a thin absorption body include an absorption sheet and a polymer sheet, and the thickness thereof is preferably from 0.3 to 5.0 mm. The absorption sheet or polymer sheet can be used without any particular limitation, as long as it is a material usually used for an absorbent article such as a sanitary napkin. Examples of the absorption sheet include absorption paper, nonwoven fabric and pulp sheet obtained by forming a fiber into a sheet with a binder or the like. Examples of the polymer sheet include fluffed pulp and a sheet obtained by mixing a particulate polymer with a fiber and forming the mixture into a sheet form. In the sheet obtained by mixing a particulate polymer with a fiber and forming the mixture into a sheet form, the particulate polymer may be dispersed either in a layer form or in a three-dimensional form. The particulate polymer used for the polymer sheet is preferably a polymer capable of absorbing and holding a liquid in an amount of 20 times or more its own weight and being gelled. Examples thereof include starch, a crosslinked carboxymethylated cellulose, a polyacrylic acid and a salt thereof, and a polyacrylate graft copolymer. Also, for sufficiently maintaining the joining strength to the embossed part, as the thermoplastic chemical fiber, a fiber having high thermal adhesiveness is preferably used. When, for example, a fiber having linear PE, low-density PE, high-density PE or the like in the sheath structure or a fiber formed by incorporating PE into PP or PET, forming a sheet therefrom and dividing the sheet into strips is added, joining by heat of embossing can be more firmly maintained.

The liquid-permeable sheet is not particularly limited, as long as it is a sheet-like material having a structure allowing for permeation of a liquid. As the material for woven or nonwoven fabric, both a natural fiber and a chemical fiber can be used. Examples of the natural fiber include a cellulose such as fluffed pulp and cotton. Examples of the chemical fiber include a regenerated cellulose such as rayon and fibril rayon, a semisynthetic cellulose such as acetate and triacetate, a thermoplastic hydrophobic chemical fiber, and a thermoplastic hydrophobic chemical fiber subjected to a hydrophilization treatment.

Examples of the thermoplastic hydrophobic chemical fiber include a single fiber such as polyethylene (PE), polypropylene (PP) and polyethylene terephthalate (PET), a fiber obtained by graft copolymerization of PE and PP, and a composite fiber such as fiber having a sheath-core structure.

As the liquid-impermeable sheet, for example, a film mainly composed of PE, PP or the like, an air-permeable resin film, a sheet obtained by joining an air-permeable resin film to a nonwoven fabric such as spunbond and spunlace, or a sheet having a multilayer structure such as three-layer structure may be suitably used. Considering flexibility not impairing the wearing feel, for example, a film mainly composed of a low-density polyethylene (LDPE) resin and having a basis weight of 15 to 30 g/m² is preferably used.

A second sheet may be disposed between the liquid-permeable sheet and the absorption body. The material and production method are not particularly limited, as long as it is employed for the permeable sheet above, but for facilitating absorption of a body fluid from the liquid-permeable sheet, a second sheet more increased in the density than the liquid-permeable sheet is preferably used.

On the non-skin-contact surface of the liquid-impermeable sheet, a slip stopper 11 for fixing to underwear such as a panty is preferably provided on the non-skin-contact surface side of the liquid-impermeable sheet. The slip stopper may be continuously coated in a line or belt fashion or may be coated intermittently. Examples of the slip stopper include a hot-melt adhesive. A hot-melt adhesive having tackiness at ordinary temperature is preferred, and examples thereof include a pressure-sensitive adhesive. The adhesive has a basis weight of 10 to 200 gsm and is coated in a uniform, striped or dot pattern by coater coating, bead coating or the like. An acrylic adhesive may be also preferably used. Other examples include a member comprising a tape part and a plurality of hook parts rising on a tape part surface.

The absorbent article of the present invention can be used as a sanitary napkin, a pantiliner, an incontinence pad or the like.

The production method of the absorbent article of the present invention includes at least an embossing step of stacking a liquid-permeable sheet 2 and an absorption body 4 and compressing the stack in the thickness direction. The compressed groove (right and left compressed grooves, front-side compressed groove, rear-side compressed groove) formed along the longitudinally-extending side edge part of the absorbent article, the transverse compressed groove and the point-like compressed part may be simultaneously formed by the embossing or may be separately formed in any order. For example, in the case of simultaneously forming these grooves and parts, between a roll (first roll) having on the roller surface thereof convex streaks corresponding to the entire shape of these compressed grooves and convex parts corresponding to the entire shape of the point-like compressed part and a smooth roll (second roll), an absorbent material after stacking a liquid-permeable sheet and an absorption body is disposed by arranging the liquid-permeable sheet to come into contact with the first roll, and the absorbent material is compressed.

Finally, a liquid-impermeable sheet 3 is supplied to the non-skin-contact surface side of the absorption body 4 of the embossed absorbent material, and the absorption body 4 and the liquid-impermeable sheet 3 are joined. The absorption body and the liquid-impermeable sheet may be joined using, for example, a hot-melt adhesive.

In the embodiment above, the production method of a sanitary napkin is described as an example, but this method can be also applied to the production of other absorbent articles, for example, a pantiliner or an incontinence pad.

### DESCRIPTION OF REFERENCE NUMERALS

- 1:: Absorbent article
- 2:: Liquid-permeable sheet
- 3:: Liquid-impermeable sheet
- 4:: Absorption body
- 5:: Center region of absorption body
- 6:: Side sheet
- 7a:: Left compressed groove
- 7b:: Right compressed groove
- 7a', 7a":: Left compressed groove
- 7b', 7b":: Right compressed groove
- 7c:: Front-side compressed groove
- 7d:: Rear-side compressed groove
- 7e, 7f:: Transverse compressed groove
- 7g:: Side edge-part compressed groove
- 8:: High-compressed part
- 8a:: Nearly transverse high-compressed part
- 8b:: Non-transverse high-compressed part
- 9:: Low-compressed part
- 10:: Point-like compressed part
- 11:: Slip stopper
- 12a:: Rear end part of front-side compressed groove
- 12b:: Front end part of left compressed groove, front end
- part: of right compressed groove
- 12c:: Rear end part of left compressed groove, rear end part of right compressed groove
- 12d:: Front end part of rear-side compressed groove
- 13:: Longitudinal centerline of compressed groove
- 14:: Intermittent part
- S:: Length of non-transverse high-compressed part in the width direction of compressed groove
- T1:: Length of nearly transverse high-compressed part in the longitudinal direction of compressed groove
- T2:: Length of non-transverse high-compressed part in the longitudinal direction of compressed groove
- Y:: Width of compressed groove where a nearly transverse high-compressed part is located
- V1, V2:: Length of compressed groove between a nearly transverse high-compressed groove and a nearly transverse high-compressed groove
- W1:: Width of compressed groove between a nearly transverse high-compressed groove and a nearly transverse high-compressed groove
- W2:: Width of compressed groove where a nearly transverse high-compressed part is located
- Z:: Length of intermittent part

## Claims

1. An absorbent article comprising at least a liquid-permeable sheet, a liquid-impermeable sheet, and an absorption body sandwiched between said liquid-permeable sheet and said liquid-impermeable sheet, which is an absorbent article where, on the skin-contact surface side, a pair of compressed grooves for integrating from the liquid-permeable sheet to the absorption body are formed along the longitudinal direction, wherein
said compressed groove consists of a high-compressed part and a low-compressed part, said high-compressed part is composed of nearly transverse high-compressed parts formed so as to almost fully traverse the compressed groove in the width direction and disposed at intervals in the longitudinal direction of the compressed groove and non-transverse high-compressed parts formed not so as to traverse the compressed groove and disposed at intervals in the region between said nearly transverse high-compressed parts, and by the arrangement of said non-transverse high-compressed parts, said low-compressed part is formed so as to continue in the longitudinal direction of the compressed groove between said nearly transverse high-compressed parts.

2. The absorbent article according to claim 1, wherein at least a part of said non-transverse high-compressed parts are provided so as to cross the longitudinal centerline of the compressed groove.

3. The absorbent article according to claim 1 or 2, wherein at least one of said nearly transverse high-compressed parts is provided so as to be symmetric with respect to the longitudinal centerline of the compressed groove.

4. The absorbent article according to any one of claims 1 to 3, wherein the distance between said nearly transverse high-compressed parts is from 10 to 50 mm.

5. The absorbent article according to any one of claims 1 to 4, wherein the width of the compressed groove in which said nearly transverse high-compressed part is disposed is larger than the width of the compressed groove in which said nearly transverse high-compressed apart is not disposed.

6. The absorbent article according to any one of claims 1 to 5, wherein the distance between said nearly transverse high-compressed part and the non-transverse high-compressed part located closest to said nearly transverse high-compressed part is larger than the distance between non-transverse high-compressed parts located between nearly transverse high-compressed parts.
